# EUROPEAN PATENT SPECIFICATION

(11) **EP 3 988 927 B1**
(45) Date of publication and mention of the grant of the patent: **13.11.2024**
(21) Application number: 21203691.7
(22) Date of filing: 20.10.2021
(51) Int. Cl.: G01N 23/2005, G01N 33/204, G01N 1/28

(54) **METHOD FOR PREPARING REFERENCE SAMPLES FOR DETERMINING RESIDUAL AUSTENITE BY X-RAY DIFFRACTION**
VERFAHREN ZUR HERSTELLUNG VON REFERENZPROBEN ZUR BESTIMMUNG VON RESTAUSTENIT DURCH RÖNTGENBEUGUNG
PROCÉDÉ DE PRÉPARATION D'ÉCHANTILLONS DE RÉFÉRENCE POUR LA DÉTERMINATION DE L'AUSTÉNITE RÉSIDUELLE PAR DIFFRACTION DES RAYONS X

(30) Priority: 22.10.2020 IT 202000025093
(43) Date of publication of application: 27.04.2022
(73) Proprietor: GNR S.r.l., 28010 Agrate Conturbia (NO) (IT)
(72) Inventor: SERALESSANDRI, Luca, I-28010 Agrate Conturbia (NO) (IT); ELIA, Alice, I-28010 Agrate Conturbia (NO) (IT)
(74) Representative: Cammareri, Emanuele

(56) References cited:
- CN-A- 104 215 489
- CN-A- 105 445 304
- MICHAELIS R E ET AL: "Standard Reference Materials A standard reference material containing nominally four percent austenite NBS SP 260-25", 1 February 1971 (1971-02-01), pages 1 - 28, XP061046739, Retrieved from the Internet <URL:https://nvlpubs.nist.gov/nistpubs/Legacy/SP/nbsspecialpublication260-25.pdf> [retrieved on 19710201], DOI: 10.6028/NBS.SP.260-25
- WOLFENDEN A ET AL: "Interlaboratory Evaluation of ASTM Practice for X-Ray Determination of Retained Austenite in Steel with Near-Random Crystallographic Orientation (E 975)", vol. 15, no. 2, 1 January 1987 (1987-01-01), US, pages 95, XP055819052, ISSN: 0090-3973, Retrieved from the Internet <URL:http://dx.doi.org/10.1520/JTE10988J> [retrieved on 20210629], DOI: 10.1520/JTE10988J

## Description

### TECHNICAL FIELD

The present invention relates to the field of material and process controls in the steel industry up to quality checks on the finished product.

### STATE OF THE ART

Steels can be characterized by the presence of several crystalline phases (such as Ferrite, Martensite and Austenite), the formation and stability of which is regulated by various factors including the cooling rate, the chemical composition and the type of processing. Austenite, in particular, is a metastable phase that forms at high temperatures; in the cooling process it transforms into a more stable crystalline phase (such as Ferrite). In industrial production processes, under certain conditions, this transformation can occur incompletely. The residual austenite is the fraction of the austenitic phase that has not transformed. For high-performance steels, the accurate measurement of the amount of Austenite is a key factor.

The determination of the amount of residual Austenite is one of the fundamental controls in the production processes of steel components, as it contributes to define their mechanical performance. Checking the content of residual Austenite allows the production of specific components for different applications.

X-ray diffraction is one of the most used techniques to carry out this check in that the analysis is non-destructive. It is therefore of considerable interest to be able to have reference samples capable of validating the results obtained with the tools based on this technique.

The first reference samples (SRM) for the determination of residual Austenite by X-ray diffraction were produced beginning in 1970 by the National Institute of Standards and Technology (NIST) by sintering austenitic and ferritic/martensitic steel powders. In parallel to the production, NIST has published procedures for the preparation and verification of SRM samples which describe the characteristics of the starting materials (particle size between 37-44 µm for ferritic powder, between 44-53 µm for austenitic powder) and the techniques used for characterization (X-ray Fluorescence and Optical

Microscopy) are described [G.E. Hicho et al, A standard reference material containing nominally four percent Austenite, Advances in Xray Analysis 14 (1970), pp. 78-91; G.E. Hicho et al, A standard reference material containing nominally four percent Austenite, NBS SP 260-25] (also published as MICHAELIS R E ET AL: "Standard Reference Materials A standard reference material containing nominally four percent austenite NBS SP 260-25", NIST, NATIONAL INSTITUTE OF STANDARDS AND TECHNOLOGY (NIST),1 February 1971 (1971-02-01), pages 1-28, XP061046739)).

The production process described in the 1970 standards is extremely complex as well as unreliable. Consider, for example, that 22 steps are proposed for the preparation of samples.

The procedure for determining residual Austenite by X-ray diffraction is described by ASTM E975 [ASTM E975-13, Standard Practice for X-Ray Determination of Retained Austenite in Steel with Near Random Crystallographic Orientation, ASTM International, West Conshohocken, PA, 2013].

The applicability of the legislation, the first version of which dates back to 1984, was assessed by means of a Round-Robin test procedure in 1987 [R.W. Hinton, Interlaboratory evaluation of ASTM practice for X-ray determination of retained Austenite in nearrandom crystallographic orientation (Practice E975), Journal of Testing and evaluation 15 (1987), pp. 95-100.]. From this study it was found that, on heat treated steel samples 20, the intra-laboratory repeatability is 3% and the interlaboratory reproducibility is 4% with a confidence interval of 95%. In the same study, the reference samples produced by NIST were also analyzed for which discrepancies between the expected values and the results obtained by applying the ASTM E975 standard were found. The authors attribute these differences to the compositional characteristics of the SRM samples (content of alloying elements greater than 15%).

The conclusions of this study were included in the current ASTM legislation, in particular in paragraph 1.5, which deals with the applicability of the legislation, and in paragraphs 6.1 and 6.2, which concern the accuracy of the results that can be obtained. Here it is also specified that it is not possible to determine the deviation of the values obtained from the real ones since currently there is no independent method to determine the content of Austenite in a reference sample.

The production and sale of NIST samples was definitively discontinued starting from 1998, probably also as a result of the study carried out by the American research laboratory Lambda Technologies. In a 2006 publication [Lambda Technologies, Diffraction Notes 33, 2006] Lambda Technologies itself mentions that the quality of the samples was not sufficient to ensure adequate accuracy in quality control processes.

### OBJECTS AND SUMMARY OF THE INVENTION

The measurements carried out by the Applicant on reference samples currently available on the market have shown a behavior similar to that found in 1987, i.e. important discrepancies (20-30%) between the results obtained by analyzing reference samples and real samples certified through the Round procedure Robin (interlaboratory).

The consistency between the results obtained on reference samples and on real samples is clearly an essential requirement for controlling the quality of a production and / or the development of new materials.

Starting from the information available on the NIST samples and from the data collected on the samples present on the market, the Applicant not only determined what are the necessary and sufficient conditions to create reference samples equivalent to real samples, but it did so using a minimum number of production steps, appropriately selecting the best grain size. The objectives of the present invention are:
1. the overcoming of what stated in point 6.2 of the ASTM E975-13 standard, that is the development of a method of preparation of reference samples that allows to know a priori the Austenite content in a clear and controlled way; and
2. the implementation of a simpler and more reliable production process than the previous ones, i.e. those published by NIST in 1970-71, thanks to the use of starting materials with the appropriate particle size and the drastic reduction of production steps from 22 to 4.

It is an object of the present invention to overcome the drawbacks of the prior art. In particular, it is an object of the present invention to define the method (laboratory materials and instrumentation) and the procedure for making homogeneous samples that can be used as a reference for the determination of residual Austenite for overcoming what is stated in Section 6.2 of ASTM E975-13.

A further object of the present invention is to have reference samples for the determination of residual Austenite by diffraction of rays that can be produced and marketed for the steel industry.

These and further objects of the present invention are achieved by means of a system incorporating the characteristics of the attached claims, which form an integral part of the present description.

The samples, object of the present invention, are prepared starting from a "quantity of the austenitic phase known a priori" or measured with independent instrumentation and more accurate and precise than that with which the sample will then be analyzed (for example a diffractometer).

"Austenitic phase quantity known a priori" means that the samples must have a precise quantity that can be obtained through "independent instrumentation" i.e., the Austenite content in the final sample is determined by a measuring instrument as and not by an inter-laboratory round-robin test.

The task of the present invention is concerned with a method for the preparation of reference samples for determining residual Austenite by X-ray as set forth in claim 1.

As regards the starting materials, these must have a certified chemical composition (for both austenitic and ferritic steel powders) and an average particle size between 1 µm and 5 µm.

The characterization of the phase purity of the starting materials is carried out by X-ray diffraction. In a preferred aspect of the invention, the analyzes are carried out with diffractometers controlled with reference standards NIST SRM 640e and 1976b.

The particle size of the starting powders and the dimensional homogeneity of the two phases surprisingly allow for a linear and direct response between the theoretical content of Austenite (weighted quantity) and the content observed by X-ray diffraction.

The expression "dimensional homogeneity of the two phases" means that the samples are prepared starting from powders with different crystalline phases. The mixture is homogeneous when the two phases are homogeneously dispersed in the sample. The evaluation is done measuring the Austenite content on the two surfaces of the sample by X-ray diffraction, according to the ASTM E975-13 standard. Three measurements are made on both surfaces; between one measurement and another the sample is rotated on itself randomly. This allows to evaluate the homogeneity of the sample.

Further characteristics and objects of the present invention will become more apparent from the following description.

### BRIEF DESCRIPTION OF THE FIGURES

The invention will be described below with reference to some examples, provided by way of non-limiting examples, and illustrated in the attached figures.

These figures illustrate different aspects and results of the present invention and, where appropriate, relevant reference numerals.
Figure 1 shows a block diagram relating to the method of the present invention.
Figure 2 shows a graph showing the Austenite value (vol%) determined by X-ray diffraction on the abscissas and the theoretical Austenite value on the ordinates, i.e. the quantity of Austenite weighed for the preparation of the reference sample.
Figure 3 shows a graph showing the perfect correlation between the reference samples prepared in the laboratory (triangular points) and the real samples (round points). The graph refers to samples prepared with iron powder with a particle size between 1 µm and 5 µm.
Figure 4 shows a graph showing the Austenite value (vol%) determined by X-ray diffraction on the abscissas and the theoretical Austenite value on the ordinates, i.e. the quantity of Austenite weighed for the preparation of the reference sample. The graph refers to samples prepared with iron powder with particle size between 6 µm and 8 µm.

### DETAILED DESCRIPTION OF THE INVENTION

While the invention is susceptible to various modifications and alternatives, some preferred embodiments are shown in the examples and will be described below in detail. It is to be understood, however, that there is no intention of limiting the invention to the specific embodiment illustrated, but, on the contrary, the invention intends to cover all modifications, and equivalents that fall within the scope of the invention as defined in the claims. The use of "for example", "etc.", "or" indicates non-exclusive alternatives without limitation unless otherwise indicated. The use of "include" means "includes, but not limited to" unless otherwise indicated.

The reference samples for determining residual Austenite by X-ray diffraction are prepared starting from ferritic and austenitic phase powders, mixed in known quantities and pressed into a pellet with the addition of a binder.

The Austenite content in the final sample is therefore known a priori because it is measured with an independent instrumentation (analytical balance). The powders used for the preparation of the samples are of known and certified composition. The crystalline structure of the starting materials is also checked by means of a powder diffractometer before preparing the samples.

The starting materials are Ferrite and austenitic steel powders. Powders of all austenitic steels, for example AISI 5 304 or AISI 316, and ferritic / martensitic, for example AISI 430 can be used. The steel powder must have a purity greater than 99%. In a preferred form of the invention, 316 L austenitic steel powder is used, having composition Fe / Cr18 / Ni10 / Mo3, and purity 99.9%.

As far as the characteristic of the iron powder is concerned, it must have a purity greater than 95%, preferably greater than or equal to 98%.

As regards steel powder, the average particle size must be between 1 µm and 5 µm, preferably between 2 µm and 4 µm. In a particularly preferred aspect of the invention, the average nominal size of the steel particles is 5 µm. As for the iron powder, the average particle size must be between 1 µm and 5 µm, preferably between 2 µm and 4 µm . The mean size of the particles or granulometry is the value of the distribution dimensional corresponding to 90% of the dust particles. The powders are certified for their chemical composition. It is particularly preferred that the average particle sizes of iron and austenite are similar.

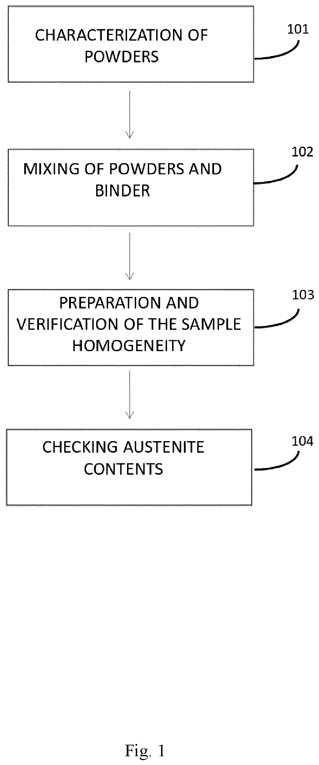

With reference to Figure 1, the first phase of procedure 101 concerns the characterization of the phase purity of the powders by X-ray diffraction. The analyzes are carried out with diffractometers controlled with NIST SRM 640e and SRM 1976b reference standards.

"Characterization of the phase purity of the powders" means that the powders used for the preparation of the samples have a chemical composition certified by the manufacturer. As for the crystalline phases present, the materials are not certified. Therefore, before preparing the samples, the powders are analyzed by X-ray diffraction. With this analysis it is verified whether the iron powder contains only the ferritic phase and whether the 316L steel powder contains only the austenitic phase. If phase impurities are present, these are quantified and considered in the calculation of the desired amount of Austenite for the reference samples.

It is therefore necessary to proceed with the mixing of the powders 102. To proceed with the mixing of the powders it is necessary to use known quantities thereof.

As regards the weighing of powders, in a preferred aspect of the invention, an analytical balance (VWR^{®}) with a resolution of 0.1 mg was used.

A third component that is mixed with the powders is a binder. The binder must be amorphous and possess excellent binding properties as well as be stable to X-rays. In a preferred aspect of the invention the wax-based binder CEREOX^{®} 10 (Fluxana^{®}) commonly used to form pellets is preferred. Generally 1 part of binder is used for 4 parts of metal powders.

In a preferred aspect of the invention, the iron powder is weighed in a nacelle and then the contents are transferred into a zirconia jar. The residual powder in the nacelle is then weighed again in order to calculate the exact amount of powder of iron used. Generally the quantity of iron powder to be weighed is between 2.0 g and 3.5 g. With the same procedure, the austenitic steel powder is weighed in a new nacelle and then the contents are transferred into the same jar containing the iron powder. Generally the quantity of iron powder to be weighed is between 0.2 g and 0.8 g. The amorphous binder is then weighed as described above.

The mixing of the powders can take place in any way known in the art. In a preferred aspect of the invention, a laboratory mill was used, preferably a vibromill. In particular, in a preferred aspect of the invention a vibromill MM400 (Retsch^{®}), equipped with zirconia jars and grinding spheres of the same material; the diameter of the spheres was 3 mm. As known to those skilled in the art, mixing times must be proportional to the amount of sample inserted in the jar. For 2-4 total grams (binder plus Iron and Austenite powders) it is advisable to mix for 4 minutes at 10 Hz.

At the end of the mixing, the grinding spheres are removed and the contents of the j ar are transferred to an airtight container on which the lot number and the Austenite content are indicated, calculated on the basis of the exact quantities of ferritic and austenitic powder inserted in the grinding jar.

The next stage involves the preparation of the sample and, in particular, the verification of its homogeneity 103.

The sample can be prepared according to any of the ways known in the art. In the present invention a mold compatible with a Vaneox^{®} 25 ton manual hydraulic press was used. (Fluxana^{®}). The amount of mixture that is used varies between between 0.5 g to 1 g. Time and pressure are characteristics well known to those skilled in the art. Generally a pressure between 2.2 ton / cm² and 8.8 ton / cm² is applied for a time between 10 and 20 seconds.

The first pellets were prepared using 1 g of powder and applying a pressure of 10 tons for 15 seconds. The press manufacturer, however, recommends a maximum pressure of 2.5 tons for samples with a diameter between 8 mm and 14 mm, as in our case (Ø 12 mm).

It was therefore decided to limit the pressure to 2.5 tons and reduce the amount of dust. The thickness of the tablets obtained with the new parameters (0.75 g, 2.5 ton, 15 seconds) is approximately 1.7 mm. Pellets prepared with 1 g of powder, 10 tons for 15 seconds have one thickness of about 2.5 mm. The maximum pressure must be reached gradually in order to reduce the air trapped in the sample, which could cause flaking of the surface layer.

Once the resulting pellet has been extracted from the mold, the tablet is applied in an amorphous support, for the protection / storage of the sample and greater ease of use by the operator.

This is analyzed by diffractometry. In a preferred aspect of the invention, an Explorer^{®} (GNR^{®}) powder diffractometer was used, equipped with both a tube with a chrome anode and a tube with a molybdenum anode. The diffractometer was checked using the NIST SRM 640 and SRM 1976 reference standards by X-ray diffraction.

The homogeneity of the tablet / sample is checked by carrying out, on both sides, three measurements along three different directions chosen at random. For each batch, a tablet is divided in half to carry out a further homogeneity check inside. The sample is defined as homogeneous if the standard deviation of all measurements is less than 1 vol%.

From the analysis of the sample, the Austenite 104 content is finally verified.

Below is an example of a preferred embodiment that will make it even clearer how the invention allows to achieve the intended objects. This example should not be construed in a limiting sense and the invention thus conceived is susceptible of numerous modifications and variations all falling within the scope of the present invention as it results from the attached claims.

### EXAMPLE ACCORDING TO THE INVENTION

### Used materials:

- Iron powder, purity ≥ 98%, average particle size between 1 µm and 5 µm (Goodfellow^{®})
- 316 L austenitic steel powder, composition Fe / Cr18 / Ni10 / Mo3, puriy 99.9%, average particle size between 1 µm and 5 µm (Goodfellow^{®})
- Cereox^{®} binder (Fluxana^{®})

### Equipment used:

- Analytical balance with resolution 0.1 mg (VWR^{®}).
- Vibromill MM400 (Retsch^{®}), equipped with zirconia jars and grinding spheres of the same material; diameter of the balls 3 mm.
- Vaneox^{®} 25 ton manual hydraulic press (Fluxana^{®}).
- Explorer^{®} (GNR^{®}) powder diffractometer, equipped with both a tube with a chrome anode and one with a molybdenum anode). The diffractometer was tested using the NIST SRM 640e and SRM 1976b reference standards.

### Procedure used:

### 1. Characterization of the materials

The iron and 316L steel powders were analyzed with a powder diffractometer using a chromium anode (Cr Kα 2.2897 Å) and subsequently with a Molybdenum anode (Mo Kα 0.7093 Å). The quantification of the phases starting from the diffractograms collected with the instrument was carried out according to the ASTM E975 standard.

### 2. Mixing of the powders

2.7015 g of iron powder were weighed into a nacelle and transferred to the zirconia jar. The empty nacelle was weighed and the weight of the powder left in the nacelle was noted (0.0104 g). The weight of the residual powder was subtracted from the weighing and the net weight of the iron powder transferred into the jar was noted (2.6911 g).

0.3006 g of 316L steel powder was weighed in a new nacelle, which was transferred to the same jar in which the iron powder was placed. As previously done, the empty nacelle was weighed and the weight of the remaining powder was noted in the nacelle (0.0097 g). Eventually the net weight of the steel powder transferred to the jar was 0.2909 g. 0.7521 g of Cereox were then weighed and placed in the grinding jar together with 2 zirconia spheres with a diameter of 3 mm.

After closing the jar, mixing was started in the vibromill for 4 minutes at 10 Hz. At the end of mixing, the grinding balls were removed and the contents of the jar were transferred into an airtight container on which it was noted the batch number and the Austenite content calculated on the basis of the exact quantities of ferritic and austenitic powder inserted in the grinding jar.

### 3. Preparation of the samples

0.7505 g of powder-binder mixture was weighed and transferred into the mold. The piston was inserted into the mold, and it was put into the press by applying a pressure of 2.5 tons for 15 seconds. The thickness of the tablets obtained with these parameters is approximately 25 1.7 mm.

### 4. Verification of samples

Once the sample has been extracted from the mold, the Austenite content is verified by analyzing each sample by X-ray diffraction. The sample is analyzed three times per side.

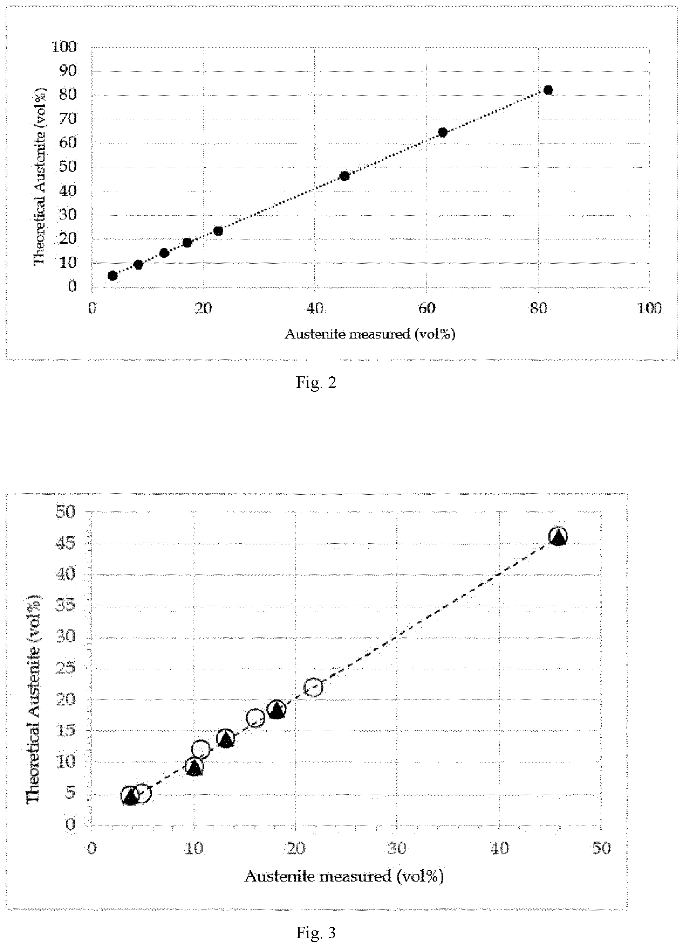

The particle size of the starting powders and the dimensional homogeneity of the two phases are the two fundamental parameters to have a linear and direct response, between the theoretical content of Austenite (weighted quantity) and the content observed by X-ray diffraction: using Austenite and Ferrite with average particle size between 1 µm and 5 µm, a linear correlation is obtained with an angular coefficient substantially equal to 1 as shown in figure 2. A linear and direct correlation is observed (y = 0.9935x + 1.4914; R² = 0.9998) by indicating that the Austenite value measured for X-ray diffraction corresponds to the expected value from the weighings and therefore no calibration between the expected value and the measured value is necessary.

The graph in figure 3 instead shows the correlation between the reference samples prepared with austenic and ferritic powder with average particle size less than or equal to 5 µm in the laboratory (triangular points) and those of real samples (round points).

This graph shows how the response of the reference samples prepared for weighing give an in-line response (y = 0.9957x + 0.4602; R2 = 0.9978) with the response of real samples prepared by heat treatment and whose theoretical value was determined through an interlaboratory round-robin procedure. This correspondence, found for X-ray diffraction measurements carried out with a Molybdenum anode, was not observable with the reference samples currently available on the market.

### COMPARATIVE EXAMPLE

By essentially repeating the example according to the invention, but using a particle size of Austenite and Ferrite greater than 5 µm, a linear response is no longer observed.

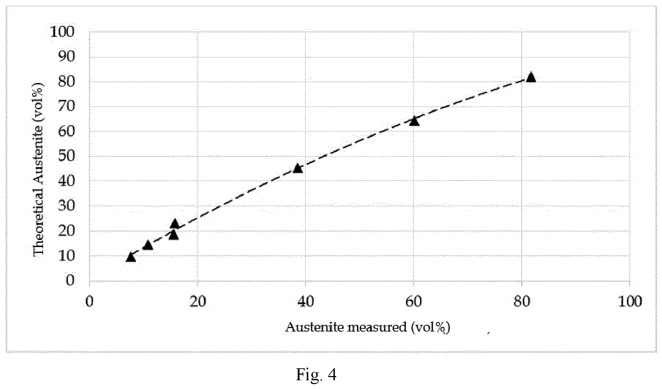

In particular, Austenite with a grain size of 5 µm and Ferrite with a grain size between 6-8 µm was used. As can be seen from Figure 4, a good correlation is obtained, but neither linear nor direct (y = -0.0038x2 + 1.3002x + 0.7994; R2 = 0.9978). A second order correlation is observed, indicating that a calibration is required between the expected Austenite value and the value measured by X-ray diffraction.

## Claims

1. Method for preparing reference samples for the determination of residual Austenite by X-Ray diffraction **characterized in that** it comprises the following steps:
a. Characterizing (101) the phase purity of iron powders having average particle size between 1 µm and 5 µm and greater than 95% purity and austenitic steel powder with average particle size between 1 µm and 5 µm and purity greater than 99% by x-ray diffraction;
b. Mixing (102) for a suitable mixing time said powders and an amorphous binder by preparing a sample, wherein the mixing time is proportional to the sum of the amounts of powders and amorphous binder which make up the sample;
c. Verifying (103) that the sample is homogeneous by performing 6 measurements of the austenitic phase by X-ray diffraction in such a way that, on both sides of the sample, three measurements are taken along three different directions chosen at random, with the sample being homogeneous if a standard deviation of the measurements is less than 1 vol% so that the sample has, between weighted Austenite content and content observed by X-ray diffraction, a linear and direct correlation with an angular coefficient substantially equal to 1; and
d. Checking (104) the content of residual Austenite in said sample by X-ray diffraction.

2. Method according to claim 1 wherein the characterization (101) of the phase purity of the starting materials by means of X-ray diffraction is carried out with a powder diffractometer equipped with either a chromium anode tube or a molybdenum anode tube using NIST reference standards SRM 640 and SRM 1976.

3. Method according to one of the preceding claims where the iron powders have purity ≥ 98% and average particle size between 2 µm and 4 µm; the austenitic steel powders have composition Fe / Cr18 / Ni10 / Mo3, purity 99.9%, and average particle size between 2 µm and 4 µm and the amorphous binder is a wax-based binder.

4. Method according to claim 1 where the mixing (102) of step b is carried out with a vibromill equipped with zirconia jars and grinding spheres of the same material, the diameter of said spheres being 3 mm.

5. Reference samples for the determination of residual Austenite by X-ray diffraction prepared with the method according to claim 1 **characterized in that** the sample is homogenous so that a standard deviation of 6 measurements of the austenitic phase by X-ray diffraction performed in such a way that, on both sides of the sample, three measurements are taken along three different directions chosen at random, is less than 1 vol%.

## Patentansprüche

1. Verfahren zur Herstellung von Referenzproben für die Bestimmung von Restaustenit durch Röntgendiffraktometrie, **dadurch gekennzeichnet, dass** es folgende Schritte umfasst:
a. Charakterisierung (101) der Phasenreinheit von Eisenpulvern mit einer mittleren Teilchengröße zwischen 1 µm und 5 µm und einer Reinheit von mehr als 95% und von austenitischen Stahlpulvern mit einer mittleren Teilchengröße zwischen 1 µm und 5 µm und einer Reinheit von mehr als 99% durch Röntgendiffraktometrie;
b. Mischen (102) der oben genannten Pulver und eines amorphen Bindemittels während einer geeigneten Mischzeit durch Herstellung einer Probe, wobei die Mischzeit proportional zur Summe der Mengen der Pulver und des amorphen Bindemittels ist, aus denen die Probe besteht;
c. Prüfung (103), ob die Probe homogen ist, indem 6 Messungen der Austenitphase durch Röntgendiffraktometrie so durchgeführt werden, dass auf jeder Seite der Probe 3 Messungen in 3 verschiedenen, zufällig ausgewählten Richtungen durchgeführt werden, wobei die Probe homogen ist, wenn die Standardabweichung der Messungen weniger als 1 Vol.% beträgt, so dass die Probe eine lineare und direkte Korrelation zwischen dem gewichteten Austenitgehalt und dem durch Röntgendiffraktometrie festgestellten Gehalt mit einem Winkelkoeffizienten von im Wesentlichen gleich 1 aufweist; und
d. Prüfen (104) des Restaustenitgehalts der oben genannten Probe durch Röntgendiffraktometrie.

2. Verfahren nach Anspruch 1, wobei die Charakterisierung (101) der Phasenreinheit der Ausgangsmaterialien durch Röntgendiffraktometrie mit einem Pulverdiffraktometer, das entweder mit einer Chromanodenröhre oder einer Molybdänanodenröhre ausgestattet ist, unter Verwendung der NiST-Referenzstandards SRM 640 und SRM 1976 durchgeführt wird.

3. Verfahren nach einem der vorhergehenden Ansprüche, wobei die Eisenpulver eine Reinheit ≥ 98 % und eine mittlere Teilchengröße zwischen 2 µm und 4 µm aufweisen; die austenitischen Stahlpulver eine Zusammensetzung Fe / Cr18 / Ni10 / Mo3, eine Reinheit von 99,9% und eine mittlere Teilchengröße zwischen 2 µm und 4 µm aufweisen; und das amorphe Bindemittel ein Bindemittel auf Wachsbasis ist.

4. Verfahren nach Anspruch 1, wobei das Mischen (102) von Schritt b mit einer Vibromühle durchgeführt wird, die mit Zirkoniumdioxidbechern und Mahlkugeln aus demselben Material ausgestattet ist, wobei der Durchmesser der Kugeln 3 mm beträgt.

5. Referenzproben für die Bestimmung von Restaustenit durch Röntgendiffraktometrie, hergestellt nach dem Verfahren gemäß Anspruch 1, **dadurch gekennzeichnet, dass** die Probe derart homogen ist, so dass eine Standardabweichung von 6 Messungen der austenitischen Phase durch Röntgendiffraktometrie, die so durchgeführt werden, dass auf jeder Seite der Probe drei Messungen in drei verschiedenen, zufällig ausgewählten Richtungen durchgeführt werden, weniger als 1% Vol. beträgt.

## Revendications

1. Procédé de préparation d'échantillons de référence pour la détermination de l'austénite résiduelle par diffraction des rayons X **caractérisé en ce qu'**il comprend les étapes suivantes :
a. Caractérisation (101) de la pureté de phase des poudres de fer dont la taille moyenne des particules est comprise entre 1 µm et 5 µm et supérieure à 95 %, ainsi que de la poudre d'acier austénitique dont la taille moyenne des particules est comprise entre 1 pm et 5 pm et supérieure à 99 % par diffraction des rayons X ;
b. Mélange (102) pendant un temps de mélange approprié desdites poudres et d'un liant amorphe par la préparation d'un échantillon, le temps de mélange étant proportionnel à la somme des quantités de poudres et de liant amorphe qui composent l'échantillon ;
c. Vérification (103) de l'homogénéité de l'échantillon en effectuant 6 mesures de la phase austénitique par diffraction des rayons X de telle sorte que, de part et d'autre de l'échantillon, trois mesures sont effectuées selon trois directions différentes choisies au hasard, l'échantillon étant homogène si l'écart type des mesures est inférieur à 1 % en volume, de telle sorte que l'échantillon présente, entre la teneur pondérée en austénite et la teneur observée par diffraction des rayons X, une corrélation linéaire et directe avec un coefficient angulaire sensiblement égal à 1 ; et
d. Contrôle (104) de la teneur en austénite résiduelle dans ledit échantillon par diffraction des rayons X.

2. Procédé selon la revendication 1, dans lequel la caractérisation (101) de la pureté de phase des matériaux de départ par diffraction des rayons X est effectuée avec un diffractomètre à poudre équipé d'un tube à anode de chrome ou d'un tube à anode de molybdène, en utilisant le matériau standard de référence NIST SRM 640 et SRM 1976.

3. Procédé selon l'une des revendications précédentes, dans lequel les poudres de fer ont une pureté > 98 % et une granulométrie moyenne comprise entre 2 µm et 4 µm ; les poudres d'acier austénitique ont une composition Fe / Cr18 / Ni10 / Mo3, une pureté de 99,9 %, et une granulométrie moyenne comprise entre 2 µm et 4 µm et le liant amorphe est un liant à base de cire.

4. Procédé selon la revendication 1, dans lequel le mélange (102) de l'étape b est réalisé avec un vibro-broyeur équipé de bols en zircone et de billes de broyage du même matériau, le diamètre de ces billes étant de 3 mm.

5. Échantillons de référence pour la détermination de l'austénite résiduelle par diffraction des rayons X préparés avec le procédé selon la revendication 1 **caractérisé en ce que** l'échantillon est homogène de telle sorte que l'écart type de 6 mesures de la phase austénitique par diffraction des rayons X effectuées de telle sorte que, de part et d'autre de l'échantillon, trois mesures sont effectuées selon trois directions différentes choisies au hasard, est inférieur à 1 % en volume.
